# EUROPEAN PATENT SPECIFICATION

(11) **EP 2 819 383 B1**
(45) Date of publication and mention of the grant of the patent: **06.02.2019**
(21) Application number: 14174168.6
(22) Date of filing: 26.06.2014
(51) Int. Cl.: H04L 29/08, H04W 4/02, A63B 24/00, A61B 5/103, A61B 5/11, A61B 5/00

(54) **User activity tracking system and device**
System zur Verfolgung von Benutzeraktivitäten und Vorrichtung
Système de suivi d'activité d'utilisateur et dispositif

(30) Priority: 28.06.2013 US 201313930347; 28.06.2013 US 201313930321; 18.06.2014 WO PCT/US2014/042839
(43) Date of publication of application: 31.12.2014
(73) Proprietor: Facebook, Inc., Menlo Park, CA 94025 (US)
(72) Inventor: Pennanen, Juho, 00500 Helsinki (FI); Kyrõlä, Aapo, Menlo Park, CA 94025 (US); Partanen, Jukka, Menlo Park, CA 94025 (US)
(74) Representative: Schröer, Gernot H.

(56) References cited:
- EP-A1- 2 457 505
- WO-A1-2012/134797
- WO-A1-2012/170449
- WO-A2-2010/096691
- US-A1- 2010 070 456
- US-B1- 8 306 514

## Description

### TECHNICAL FIELD

The present disclosure generally relates to tracking, recording and analyzing user activities, and more specifically to systems and associated methods for identifying user activities based on sensor data collected by a mobile communication device of a user. Moreover, aspects of the disclosure are also directed to software products recorded on non-transitory machine-readable data storage media, wherein such software products are executable upon computing hardware, to implement the methods of the disclosure.

### BACKGROUND

Tracking devices exist that sense and track user activities, especially sports activities. An example of a known activity tracking device is a wearable wristwatch device with a GPS receiver for tracking and analyzing 'running' activity of an associated user. Another example is a mobile application that utilizes GPS system of a respective mobile phone for recording movement of users while they exercise. Another example is a step counter used in shoes or attached to the user's clothes to collect the number of steps taken by the user. However, none of the existing tracking devices automatically sense, record, analyze and identify all types of user activities such as walking, running, jogging, cycling, rowing, driving with car, moving with bus, moving with train, walking stairs, running stairs, jumping, swimming, playing football, and skiing.

Nowadays, smartphones are equipped with an increasing number of sensors such as Global Positioning System (GPS) receivers, accelerometers, and proximity sensors, and smartphone users may find it interesting to have mobile applications that can automatically record, sense, analyze, and identify their activities. However, one of the key challenges in the automatic tracking of users' movements for the purpose of analyzing the type of activity is the classification of activity types. For example walking vs running activity may have only small difference in respect to the collected sensor data. Moreover, for the same activity, the sensor data may vary depending on how the smart phone is carried by the user. For example, the smartphone may be carried by the user in his/her hand, or in a pocket or in a backpack.

[**3^{bis}**] Document US 8,306,514 B1 describes a device to automatically provide differing information levels according to a predetermined social hierarchy includes a memory and a processor. The memory stores social templates corresponding to unique social signatures and being selectable to provide, for each level of the predetermined social hierarchy, a corresponding differing amount of information to each member of the predetermined social hierarchy and/or a social networking service. The processor receives sensor data from a sensor set which detects sensor data related to an environment of a communication device, creates a detected social signature from the received sensor data, determines which of the social signatures of the social templates has the greatest correspondence with the created social signature, retrieves from the memory the determined one social template having the greatest correspondence, and provides only as much information as allowed in the retrieved social template.

[**3^{ter}**[ Document WO 2012/134797 A1 describes a methods and apparatuses that may be used to obtain a plurality of time-referenced features extracted from signals received from one or more inertial sensors of a mobile device, wherein the time referenced features are provided according to heterogeneous time scales. The time-referenced features are combined over a decision window to infer an activity of a user co-located with the mobile device.

In view of the above, there exists a need for an activity tracking solution that accurately senses and analyzes all kinds of user activities and that addresses the limitations of existing activity tracking solutions.

### SUMMARY OF PARTICULAR EMBODIMENTS

The present disclosure seeks to provide a system, method, storage media, and device enabling tracking and recording movements of a mobile communication device. This in particular is obtained by the features of claims 1, 10, 11 and 13. Embodiments result from the dependent claims and also from the following description.

In one aspect, embodiments of the present disclosure provide a system for tracking and recording movements of a mobile communication device that includes one or more movement sensors and a wireless interface. The system includes a communication network for communicating with the mobile communication device and computing hardware for processing data supplied in operation from the mobile communication device. The mobile communication device communicates sensor signals, for example in a form of sensor data, to the system, wherein the sensor signals are indicative of motion associated with activities to which the mobile communication device is exposed by its user.

In particular embodiments, the computing hardware executes software products for analyzing the sensor signals to classify them into one or more temporal zones, and for analyzing the signals within each given temporal zone to determine one or more most likely activity types associated with the given temporal zone. The computing hardware further sends information indicating the most likely activity types associated with the temporal zones to the mobile communication device. The mobile communication device then requests its user to provide a confirmation whether or not the information indicating the most likely activity types associated with a temporal zone represents a correct analysis, and then communicates the confirmation back to the computing hardware for amending parameters and/or algorithms employed in the software products, which execute analysis of the sensor signals to improve their accuracy.

In particular embodiments, the computing hardware executes software products for analyzing the sensor signals to pre-classify the sensor signals to generate intermediate data. The intermediate data is thereafter processed in one or more processors to generate indications of likely activities associated with the sensor signals. The computing hardware further computes an aggregate of the indications to provide an analysis of one or more activities associated with the sensor signals, and then sends information indicating most likely activity types to the mobile communication device.

The processors are configured to process the sensor signals substantially in parallel, wherein the processors are mutually specialized in identifying characteristics of the signals corresponding to activities to which the processors are dedicated.

The system generates a temporal log of activities experienced by the mobile communication device, and presents the activities on a graphical user interface of a user in a timeline format.

The mobile communication device is implemented by way of at least one of: a portable computer such as laptop, a smartphone, a wrist-worn phone, a phablet, a mobile telephone, a tablet computer, a portable media device or any other computing device that can be worn by the user and is capable of processing and displaying data. Further, one or more sensors of the mobile communication device are implemented using at least one of: a gyroscopic angular sensor, an accelerometer, a GPS position sensor, cellular positioning sensor, a magnetometer, a microphone, a camera, a temperature sensor. The term cellular positioning sensor can refer to determining the location and movement of the mobile communication device can be derived/analyzed/measured using information related to a cellular network and information related to radio base stations and their signals.

When executed on the computing hardware, the software products are operable to implement supervised or semisupervised classification algorithms such as neural networks, decision forest, and support vector machines, for analysis of information included in the sensor signals. As input, the supervised or semisupervised classification algorithms can use, for instance, the amplitudes of the frequency components of the information included in the one or more sensor signals, and the output of the classification algorithms are estimated probabilities of different activities, conditional on the sensor signals.

In another aspect, the mobile communication device includes a data processor for executing a mobile software application thereat, wherein the mobile software application is operable when executed to cause a graphical user interface of the mobile communication device to present analyzed activity results provided from the computing hardware in a form of a timeline, wherein different analyzed activities are represented by mutually different symbols in respect of the timeline.

In yet another aspect, embodiments of the present disclosure provide a method of using the system for tracking and recording the movements of the mobile communication device.

In yet another aspect, embodiments of the present disclosure provide a mobile communication device for implementing the system for tracking and recording movements of the user.

In yet another aspect, embodiments of the present disclosure provide a software product recorded on a non-transitory machine-readable data storage media, such that the software product is executable upon computing hardware for implementing the method of using the system for tracking and recording movements of the mobile communication device. The software product is downloadable from a software application store to the mobile communication device.

Embodiments of the present disclosure sense, analyze and identify all types of user activities by analyzing the data collected from one or more sensors of a mobile communication device of a user. The sensor data is processed by a set of independent instances of classification algorithms and each instance is optionally dedicated to identify a specific type of activity. The output of the set of classification algorithm instances is aggregated and analyzed to generate most likely user activities associated with the mobile communication device. The identified activities are displayed on a graphical user interface of the mobile communication device in a timeline format. If the user disagrees/agrees with an identified activity, then they may provide their feedback and the feedback may be used to improve the analysis and identification of the activities for the next time. Thus, the accuracy of the analysis and identification of the user activities is optimized over time.

Alternatively embodiments of the present disclosure accurately sense, analyze and identify user activities by analyzing data collected from one or more sensors of a mobile communication device of a user. The sensor data is processed by a set of parallel processors, wherein the parallel processors are parallel instances of classification algorithms and each processor is optionally dedicated to identify a specific type of activity. The output of the set of parallel processors is aggregated and analyzed to generate most likely user activities associated with the mobile communication device. The identified activities are then displayed on a graphical user interface of the mobile communication device in a timeline format. In current disclosure parallel processors can refer to implementation architecture where part of the software is executed in different central processing units (i.e. microprocessors) and/or parallel instances of classification algorithms i.e. in parallel software processes. Parallel can refer to calculation processes executed substantially at the same time but is not limited to such a method. Executing of instances can take place one by one or as a combination of some processes executed substantially at the same time and some processes executed one by one.

Additional aspects, advantages, features and objects of the present disclosure would be made apparent from the drawings and the detailed description of the illustrative embodiments construed in conjunction with the appended claims that follow.

It will be appreciated that features of the invention are susceptible to being combined in various combinations without departing from the scope of the invention as defined by the appended claims.

Embodiments according to the invention are in particular disclosed in the attached claims directed to a method, a storage medium and a system, wherein any feature mentioned in one claim category, e.g. method, can be claimed in another claim category, e.g. system, as well. The dependencies or references back in the attached claims are chosen for formal reasons only. However any subject matter resulting from a deliberate reference back to any previous claims (in particular multiple dependencies) can be claimed as well, so that any combination of claims and the features thereof is disclosed and can be claimed regardless of the dependencies chosen in the attached claims.

In an embodiment according to the invention a method of using a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, characterized in that the method includes:
operating the at least one mobile communication device to communicate one or more sensor signals to the system, wherein the one or more sensor signals are indicative of motion associated with activities to which the at least one mobile communication device is exposed by its user;
operating the computing hardware of the system to execute one or more software products for analyzing the one or more sensor signals, wherein the computing hardware is operable to pre-classify the one or more sensor signals to generate intermediate data, and the intermediate data is thereafter processed in one or more processors to generate one or more indications of likely activities associated with the one or more sensor signals, and the computing hardware is operable to compute an aggregate of the one or more indications to provide an analysis of one or more activities associated with the one or more signals; and
operating the computing hardware to send information indicating one or more most likely activity types to the at least one mobile communication device.

In a further embodiment according to the invention, the method can be characterized in that the method further includes:
operating the computing hardware of the system to execute one or more software products for analyzing the one or more sensor signals to classifying them into one or more temporal zones, and for analyzing the one or more sensor signals within each given temporal zone to determine a most likely activity type associated with the given temporal zone;
operating the computing hardware to send information indicating one or more most likely activity types associated with the one or more temporal zones to the at least one mobile communication device; and
operating the at least one mobile communication device to request its user to provide a confirmation whether or not the information indicating the one or more most likely activity types associated with the one or more temporal zones represent a correct analysis, and to communicate the confirmation back to the computing hardware for amending parameters and/or algorithms employed in the one or more software products which execute analysis of the one or more sensor signals to improve their accuracy.

In a further embodiment according to the invention, the method can be characterized in that the method includes configuring the one or more processors to process the one or more sensor signals substantially in parallel, wherein the one or more processors are mutually specialized in identifying characteristics of the one or more signals corresponding to one or more activities to which the one or more processors are dedicated.

In a further embodiment according to the invention, the method can be characterized in that the method includes operating the system to generate a temporal log of activities experienced by the at least one mobile communication device.

In a further embodiment according to the invention, the method can be characterized in that the method includes implementing at least one mobile communication device by way of at least one of: a personal computer, a portable media device, a smart phone, a wrist-worn phone, a phablet, a mobile telephone, a tablet computer.

In a further embodiment according to the invention, the method can be characterized in that the method includes implementing the one or more sensors of the at least mobile communication device using at least one of: a gyroscopic angular sensor, an accelerometer, a GPS position sensor, a cellular position sensor, a magnetometer, a microphone, a camera, a temperature sensor.

In a further embodiment according to the invention, the method can be characterized in that the one or more software products are operable to implement when executed on the computing hardware at least one of:
a supervised or semisupervised classification analysis of information included in the one or sensor signals; and
a heuristics analysis of information included in the one or more sensor signals.

In a further embodiment according to the invention, the method can be characterized in that the supervised or semisupervised classification algorithms can use as input the amplitudes of the frequency components of the information included in the one or more sensor signals, and the output of the classification algorithms are estimated probabilities of different activities, conditional on the sensor signals.

In a further embodiment according to the invention, the method can be characterized in that the method includes arranging for the at least one mobile communication device to include a data processor for executing at least one mobile software application thereat, wherein the at least one mobile software application is operable to cause a graphical user interface of the at least one mobile communication device to present analyzed activity results provided from the computing hardware in a form of at least one timeline, wherein different analyzed activities are represented by a plurality of mutually different symbols in respect of the at least one time line.

In a further embodiment according to the invention, which can be claimed as well, a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, can be characterized in that
the at least one mobile communication device is operable to communicate one or more sensor signals to the system, wherein the one or more sensor signals are indicative of motion associated with activities to which the at least one mobile communication device is exposed by its user;
the computing hardware of the system is operable to execute one or more software products for analyzing the one or more sensor signals, wherein the computing hardware is operable to pre-classify the one or more sensor signals to generate intermediate data, and the intermediate data is thereafter processed in one or more processors to generate one or more indications of likely activities associated with the one or more sensor signals, and the computing hardware is operable to compute an aggregate of the one or more indications to provide an analysis of one or more activities associated with the one or more signals; and
the computing hardware is operable to send information indicating one or more most likely activity types to the at least one mobile communication device.

In a further embodiment according to the invention, a system can further be characterized in that
the computing hardware of the system is operable to execute one or more software products for analyzing the one or more sensor signals to classifying them into one or more temporal zones, and for analyzing the one or more sensor signals within each given temporal zone to determine a most likely activity type associated with the given temporal zone;
the computing hardware is operable to send information indicating one or more most likely activity types associated with the one or more temporal zones to the at least one mobile communication device; and
the at least one mobile communication device is operable to request its user to provide a confirmation whether or not the information indicating the one or more most likely activity types associated with the one or more temporal zones represent a correct analysis, and to communicate the confirmation back to the computing hardware for amending parameters and/or algorithms employed in the one or more software products which execute analysis of the one or more sensor signals to improve their accuracy.

In a further embodiment according to the invention, a system can further be characterized in that the one or more processors are configured to process the one or more sensor signals substantially in parallel, wherein the one or more processors are mutually specialized in identifying characteristics of the one or more signals corresponding to one or more activities to which the one or more processors are dedicated.

In a further embodiment according to the invention, a system can further be characterized in that the system is operable to generate a temporal log of activities experienced by the at least one mobile communication device.

In a further embodiment according to the invention, a system can further be characterized in that the at least one mobile communication device is implemented by way of at least one of: a personal computer, a portable media device, a smart phone, a wrist-worn phone, a phablet, a mobile telephone, a tablet computer.

In a further embodiment according to the invention, a system can further be characterized in that one or more sensors of the at least mobile communication device are implemented using at least one of: a gyroscopic angular sensor, an accelerometer, a GPS position sensor, cellular positioning sensor, a magnetometer, a microphone, a camera, a temperature sensor.

In a further embodiment according to the invention, a system can further be characterized in that the one or more software products are operable to implement when executed on the computing hardware at least one of:
a supervised or semisupervised classification analysis of information included in the one or sensor signals; and
a heuristic analysis of information included in the one or more sensor signals.

In a further embodiment according to the invention, a system can further be characterized in that the supervised or semisupervised classification algorithms can use as input the amplitudes of the frequency components of the information included in the one or more sensor signals, and the output of the classification algorithms are estimated probabilities of different activities, conditional on the sensor signals.

In a further embodiment according to the invention, a system can further be characterized in that the at least one mobile communication device includes a data processor for executing at least one mobile software application thereat, wherein the at least one mobile software application is operable to cause a graphical user interface of the at least one mobile communication device to present analyzed activity results provided from the computing hardware in a form of at least one timeline, wherein different analyzed activities are represented by a plurality of mutually different symbols in respect of the at least one timeline.

In a further embodiment according to the invention, which can be claimed as well, a software product stored on non-transitory machine-readable data storage media, can be characterized in that the software product is executable upon computing hardware of a mobile communication device for implementing a method according to the invention or any of the above mentioned embodiments.

In a further embodiment according to the invention, a software product can be characterized in that the software product is downloadable from a software application store to a mobile communication device.

A further embodiment according to the invention, which can be claimed as well, can be a mobile communication device for use when implementing the system as according to the invention or any of the above mentioned embodiments.

A further embodiment according to the invention can be a mobile communication configured to be implemented in the system as mentioned above and in use for executing a method as mentioned above.

In a further embodiment according to the invention, one or more computer-readable non-transitory storage media embody software that is operable when executed to perform a method according to the invention or any of the above mentioned embodiments.

In a further embodiment according to the invention, a system for tracking and recording movements of at least one mobile communication device including one or more movement sensors and a wireless interface, wherein the system includes a communication network for communicating with the at least one mobile communication device and computing hardware for processing data supplied in operation from the at least one mobile communication device, comprises: one or more processors; and at least one memory coupled to the processors and comprising instructions executable by the processors, the processors operable when executing the instructions to perform a method according to the invention or any of the above mentioned embodiments.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the invention is not limited to specific methods and instrumentalities disclosed herein. Moreover, those in the art will understand that the drawings are not to scale. Wherever possible, like elements have been indicated by identical numbers.
- Fig. 1: is an illustration of a high-level architecture of a system that is suitable for practicing various implementations of the present disclosure;
- Fig. 2: is an illustration of an exchange of information between a mobile communication device and a computing hardware of Fig. 1, in accordance with the present disclosure;
- Fig. 3: is an illustration of a graphical user interface (GUI) of the mobile communication device, in accordance with the present disclosure;
- Fig. 4: is an illustration of an alternative layout of the GUI of the mobile communication device, in accordance with the present disclosure;
- Fig. 5: is an illustration of steps of a method of determining activities of a user of a mobile communication device, in accordance with the present disclosure;
- Fig. 6: is an illustration of steps of using a system for tracking and recording movements of the mobile communication device, in accordance with the present disclosure;
- Fig.7: is an illustration of a graphical user interface (GUI) of the mobile communication device, in accordance with the present disclosure;
- Fig.8: is an illustration of an activity analysis system, in accordance with the present disclosure;
- Fig.9: is an illustration of steps of a method for identifying a 'cycling' activity of a user, in accordance with an embodiment of the present disclosure; and
- Fig.10: is an illustration of steps of using a system for tracking and recording movements of the mobile communication device, in accordance with the present disclosure.

### DESCRIPTION OF EXAMPLE EMBODIMENTS

The following detailed description illustrates embodiments of the disclosure and ways in which it can be implemented. Although the best mode of carrying out the invention has been disclosed, those in the art would recognize that other embodiments for carrying out or practicing the invention are also possible.

The present disclosure provides a system for tracking and recording movements of a mobile communication device that includes one or more movement sensors and a wireless interface. The system includes a communication network for communicating with the mobile communication device and computing hardware for processing data supplied in operation from the mobile communication device. The mobile communication device communicates one or more sensor signals to the system, wherein the sensor signals are indicative of motion associated with activities to which the mobile communication device is exposed by its user.

The computing hardware executes one or more software products for analyzing the sensor signals to classify them into one or more temporal zones, and for analyzing the signals within each given temporal zone to determine the most likely activity type associated with the given temporal zone. The computing hardware further sends information indicating the most likely activity types associated with the temporal zones to the mobile communication device. The mobile communication device then requests its user to provide a confirmation whether or not the information indicating the most likely activity types associated with the temporal zones represent a correct analysis, and then communicates the confirmation back to the computing hardware for amending parameters and/or algorithms employed in the software products that execute analysis of the sensor signals to improve their accuracy.

The computing hardware executes one or more software products for analyzing the sensor signals to pre-classify the sensor signals to generate intermediate data, and the intermediate data is thereafter processed in one or more processors to generate one or more indications of likely activities associated with the sensor signals. The computing hardware further computes an aggregate of the indications to provide an analysis of one or more activities associated with the sensor signals, and sends information indicating most likely activity types to the mobile communication device.

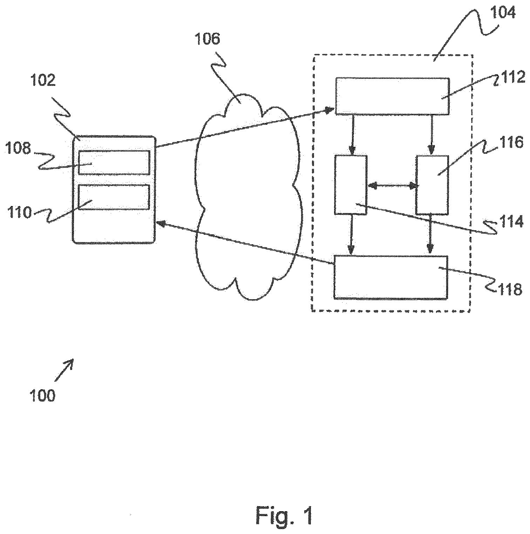

Referring now to the drawings, particularly by their reference numbers, **Fig. 1** is an illustration of a high-level architecture of a system **100** that is suitable for practicing various implementations of the present disclosure.

The system **100** includes a mobile communication device **102**, and a server system **104** coupled in communication to the mobile communication device **102** by way of a communication network **106**. The mobile communication device **102** is a handheld device of a user, and examples of the mobile communication device **102**, include, but are not limited to, a smart phone, a wrist-worn phone, a phablet, a mobile telephone, a tablet computer executing operating systems such as Android, Windows, and iOS. The server system **104** includes a computing hardware that executes one or more software products for processing data supplied in operation from the mobile communication device **102**. The server system **104** can be arranged as a cloud service or as dedicated servers located in a single site or at a plurality of mutually spatially distributed sites. Moreover, examples of the communication network **106** include, but are not limited to, a telecommunication network, and a WIFI network.

The mobile communication device **102** includes one or more sensors **108** and one or more positioning systems **110** to determine the position, movement, acceleration and/or environment of the mobile communication device **102**, when a corresponding user performs one or more activities while carrying the device **102**. Examples of such one or more activities, include, but are not limited to, walking, running, jogging, cycling, rowing, driving a car, moving with bus, moving with train, walking stairs, running stairs, jumping, swimming, playing football, and skiing. An example of the sensor **108** includes a motion sensor configured to measure the acceleration of the mobile communication device **102** in xyz-directions of a Cartesian co-ordinate system. Further examples of the sensor **108** include a gyroscopic angular sensor, a magnetometer, a microphone, a camera, and a temperature sensor. The positioning systems **110** are configured to determine the position of the mobile communication device **102** by implementing at least one of GPS positioning system, cell tower information for cellular networks, indoor positioning systems, WIFI fingerprinting and proximal WiFi networks. In an embodiment of the present invention, the mobile communication device **102** may periodically send the information collected by the sensors **108** and the positioning systems **110** to the server system **104** via the communication network **106**.

The server system **104** includes a receiving module **112**, a first processing module **114**, a second processing module **116**, and an output module **118**. The receiving module **112** receives sensor and positioning data from the mobile communication device **102**. The first processing module **114** executes a first process to analyze sensor data collected from the sensors **108**, and the second processing module **116** executes a second process to analyze positioning data collected from the positioning systems **110**. In an embodiment of the present disclosure, the first and second processes are parallel processes that might communicate with each other and also exchange data for analysis purposes. Based on the sensor data, the first processing module **114** generates an activity type of the user, and based on the positioning data, the second processing module **116** generates location and movement information pertaining to the activity. The output module **118** processes the activity type information and movement/location information of the activity to generate a summary/schedule of activities of the user. The output module **118** then sends the summary of activities to the mobile communication device **102** over the communication network **106**.

The mobile communication device **102** includes a data processor (not shown) for executing a mobile software application thereat, wherein the mobile software application is operable when executed to cause a graphical user interface (GUI) of the mobile communication device to present summary of activities provided from the server system **104** in a timeline format. The user may send their positive/negative feedback on the summary of activities to the server system **104** and the server system **104** may receive, store and implement the feedback for improving their activity analysis.

In an embodiment of the present invention, some or all of the steps/analysis in the server system **104** may be implemented in the mobile communication device **102** based on the computing resources available in the mobile communication device **102**.

Fig. 1 is merely an example, which should not unduly limit the scope of the claims herein. One of ordinary skill in the art would recognize many variations, alternatives, and modifications of embodiments herein.

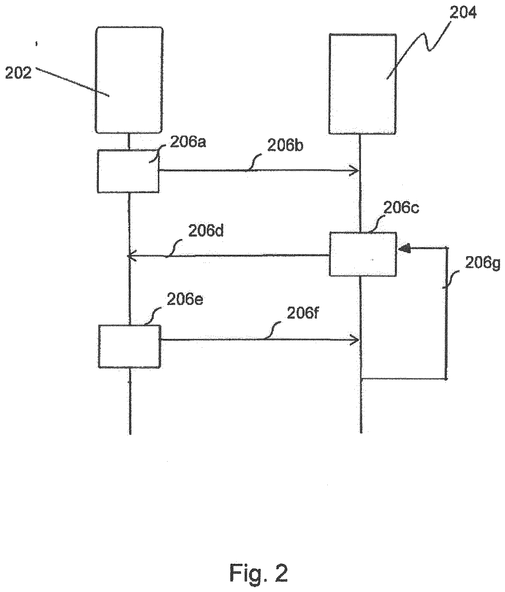

**Fig. 2** is an illustration of an exchange of information between a mobile communication device **202**, and a server system **204** for tracking and analyzing activities of a user of the mobile communication device **202**, in accordance with the present disclosure. The mobile communication device **202** and the server system **204** are examples of the mobile communication device **102** and the server system **104** respectively, and have been explained in conjunction with Fig. 1. A step **206a** takes place at the mobile communication device **202**, where the corresponding sensors and positioning systems measure sensor and positioning data when a corresponding user performs an activity. A step **206b** represents transfer of sensor and positioning data from the mobile communication device **202** to the server system **204** for analysis of the user activity. In an embodiment, the mobile communication device **202** may send the sensor and positioning data continuously or in a buffered format. In another embodiment, the mobile communication device **202** may send the sensor and positioning data to the server system **204** in a raw format. In yet another embodiment, the mobile communication device **202** may process sensor and positioning data before sending to the server system **204**. A step **206c** takes place at the server system **204**, where the server system **204** analyses the sensor and positioning data received from the device **202**. In an embodiment of the present invention, the server system **204** may perform analysis based on a supervised classification and/or other machine learning algorithms and form a summary of activities of the user. A step **206d** represents communication of summary of activities from the server system **204** to the mobile communication device **202**. A step **206e** takes place at the mobile communication device **202**, where the summary of activities is displayed on a graphical user interface (GUI) of the mobile communication device **202**. A step **206f** represents a transfer of positive/negative feedback of the user on the summary of activities to the server system **204**. Lastly, a step **206g** represents implementation of the feedback by the server system **204** by modifying parameters and/or to selecting training data for the machine learning algorithms for providing a more accurate summary of activities in future. Examples of machine learning algorithms, for activity monitoring include, but are not limited to, supervised or semisupervised classification algorithms such as neural networks, decision forest, and support vector machines. The feedback is provided as an input to the machine learning algorithms and is used to modify parameters and select training data for the machine learning algorithms.

Fig. 2 is merely an example, which should not unduly limit the scope of the claims herein. One of ordinary skill in the art would recognize many variations, alternatives, and modifications of embodiments herein.

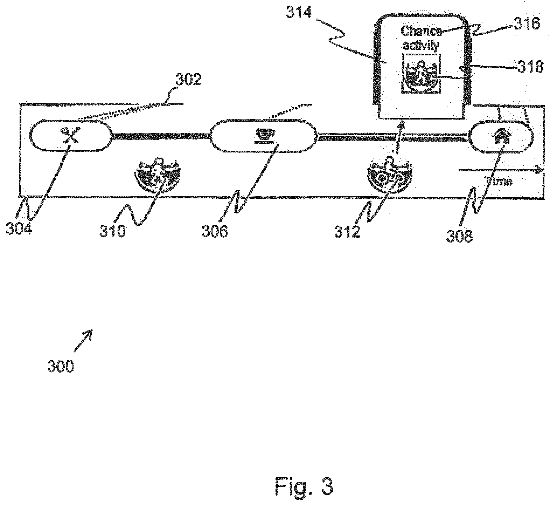

**Fig. 3** is an illustration of a graphical user interface (GUI) **302** of the mobile communication device **200**, in accordance with the present disclosure, and has been explained in conjunction with Fig. 2.

The GUI **302** displays the summary of activities of a user (received from the server system **204** at the step **206d**) in a time line format. In an exemplary embodiment, the GUI **302** displays location of the user by three location symbols, i.e. a restaurant symbol **304**, a cafeteria symbol **306** and a home symbol **308**, indicating that the user has visited the restaurant, cafeteria and home one after the other. The locations represented by the graphical symbols **304**, **306** and **308** are identified based on the GPS system of the mobile communication device **202**, cell tower information for cellular networks, indoor positioning systems, proximal WiFi, and/or WiFi fingerprinting. The GUI **302** further displays activities performed by the user by respective graphical symbols. In an example, a 'walking' activity symbol **310** is displayed between the restaurant **304** and cafeteria **306** to indicate that the user has walked from the restaurant to cafeteria. Further, a 'cycling' activity symbol **312** is displayed between the cafeteria **306** and the home **308** to indicate that the user has cycled from the cafeteria to home.

The story line display **302** may be a touch screen display and may be configured in a manner such that when a user touches or points an activity indication symbol such as the 'cycling' activity symbol **312**, or a line above or an area close to the symbol **312**, a pop-up menu **314** is presented. The pop-up menu **314** includes a text field **316** for enabling the user to modify the activity and a symbol field **318** for enabling the user to modify the graphical symbol pertaining to the activity. Similarly when a user touches or points a location symbol such as the 'restaurant' symbol **304**, or a line above or an area close to the symbol **304**, a pop-up menu (not shown) may be presented to modify the location. The modification of location/activity symbol by the user is communicated to the server system **204** as a feedback (step **206f**).

Fig. 3 is merely an example, which should not unduly limit the scope of the claims herein. One of ordinary skill in the art would recognize many variations, alternatives, and modifications of embodiments herein.

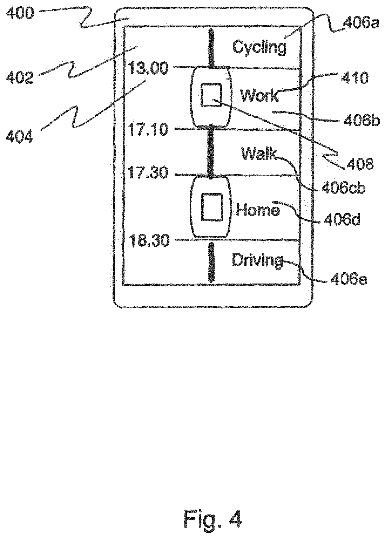

**Fig. 4** is an illustration of a graphical user interface (GUI) **402** of a mobile communication device **400**, which is an example of the mobile communication device **200**, and has been explained in conjunction with Figs. 2 and 3. The GUI **402** is an alternate layout of the GUI **302**. The GUI **402** displays a time line **404** that is divided into 'activity' zones/periods **406a**, **406b**, **406c**, **406d** and **406e**, hereinafter collectively referred to as activity zones **406**, based on start and end time of one or more activities. Each activity zone **406** illustrates an activity and corresponding location of the activity. Further, each activity zone **406** may be illustrated by a graphical symbol **408** or a text description **410** of the corresponding activity.

In an exemplary embodiment, the timeline **404** indicates that at 13:00 pm, a 'cycling' activity of user ends and he/she is stationed at a 'workplace' until 17:10 pm; at 17:10 pm, the user starts a 'walking' activity towards home; at 17:30 pm, the user reaches home and is at home until 18:30 pm; at 18:30 pm, the user starts a 'driving' activity.

Fig. 4 is merely an example, which should not unduly limit the scope of the claims herein. One of ordinary skill in the art would recognize many variations, alternatives, and modifications of embodiments herein.

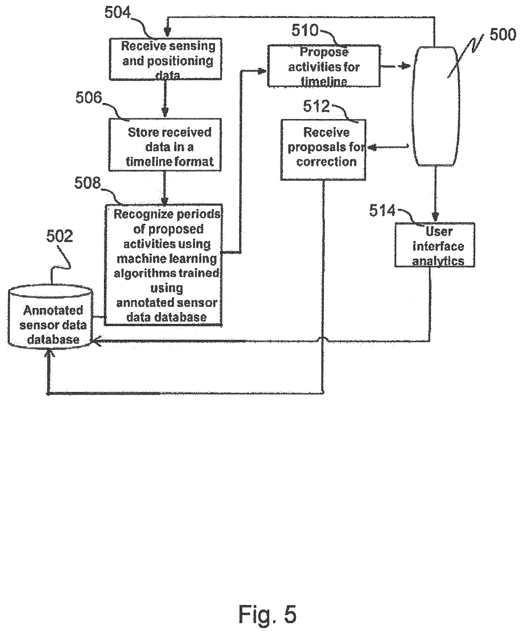

**Fig. 5** is an illustration of steps of a method of determining activities of a user of a mobile communication device **500**, in accordance with the present disclosure. The user activities are determined by an analysis logic module (not shown) which is supplied with the sensor and positioning data generated in operation by the mobile communication device **500**, and an annotated sensor data database **502**. The analysis logic module is communicatively coupled to the annotated sensor data database **502**. In an embodiment of the present invention, the analysis logic module and the annotated sensor data database **502** may be present inside a remote server system. In another embodiment of the present invention, the analysis logic module and the annotated sensor data database **502** may be present inside the mobile communication device **500** itself.

At a step **504**, the analysis logic module receives sensor and positioning data of the user from sensor and positioning systems of the mobile communication device **500**. At a step **506**, the analysis logic module stores the sensor and positioning data in a time line format in the annotated sensor data database **502**, i.e. the sensor and positioning data is time stamped to record time and date of each or some of the collected data items. The annotated sensor data database **502** includes sensor and positioning data received from the mobile communication device **500**, annotated with the information from user feedback on corresponding previously identified activities.

At a step **508**, the analysis logic module analyzes the time line and delineates one or more activity periods and recognizes user activities in each activity period using different types of machine learning algorithms, which are trained using the data stored in the annotated sensor data database **502**. For accurately identifying the activities, the analysis logic module may classify the annotated sensor and positioning data based on type of user, type of mobile device, type of user group, type of mobile device group, and demographic factors, etc. to take into account of environments and individual characteristics of the users. For example, acceleration data sensed by an accelerometer of the mobile communication device **500** for an active athletics specialized in long distance running may be different from the acceleration data sensed for a casual runner running very seldom. The active athletics may have longer steps with more predictable frequency between steps with lower variation of the frequency, than the casual runners. Therefore, for similar sensed data, the activity type may differ based on type of user group. Further, some mobile communication devices might have higher accuracy than others, therefore, for similar sensed data, the activity type may differ based on type of mobile communication device. The demographic classification may refer to segmenting users based on their gender, age, place of living etc, as the sensor data for a same activity might vary from person to person. The analysis logic module may further weigh the classified sensor data to take into consideration user specific features i.e. data could have weighted element consisting of average data from all users and user specific element from certain user based on the usage history.

At a step **510**, the analysis logic module may use the classified sensor data to make a time line of the user activities, and communicate the time line to the mobile communication device **500**. The mobile communication device **500** may display the time line on a user interface of the device **500**. At a step **512**, the analysis logic module may receive proposals for correcting one or more user activities in the timeline, from the mobile communication device **500**. The proposals for correcting the user activities may be provided to the annotated sensor data database **502** for training of the machine learning algorithms.

At a step **514**, the mobile communication device **500** may monitor whether or not the proposed time line has been viewed by the user using user interface analytics. If the user has viewed the proposed time line but not provided any correction in any activity of the timeline, then no feedback may be considered as positive feedback and is updated in the annotated sensor data database **502** that the analysis of one or more activities was correct and may be used as a training data for the algorithms. Further, if the user has viewed a storyline/timeline of a certain day and has been active in making corrections, one can conclude that that the recognized activities, which the user has not changed in the storyline, are probably considered to be correct and may be used as a training data for the algorithms. Further, it can be concluded that the recognized activities, which the user has changed in the storyline, are probably not correct. The amount of corrections made by users to certain activity can be used as a measure of recognition accuracy in regard to that activity. In the annotated sensor data database **502**, the sensor data may be labeled, with a certain confidence of the labeling, based on the annotations containing information about user feedback on the corresponding proposed activities.

In an embodiment of the present invention, the analysis logic module may implement a first machine learning algorithm for determining user activities based on the sensor and positioning data, and newly introduce a second machine learning algorithm. However, if the data analyzed based on the second algorithm is more accurate than the data analyzed by the first algorithm, then the second algorithm may be determined to be priority algorithm over the first algorithm. This feature enables to test different setups and get "votes" on the capability of a machine learning algorithm.

It should be noted here that the steps **504** to **514** are only illustrative and other alternatives can also be provided where one or more steps are added, one or more steps are removed, or one or more steps are provided in a different sequence without departing from the scope of the claims herein.

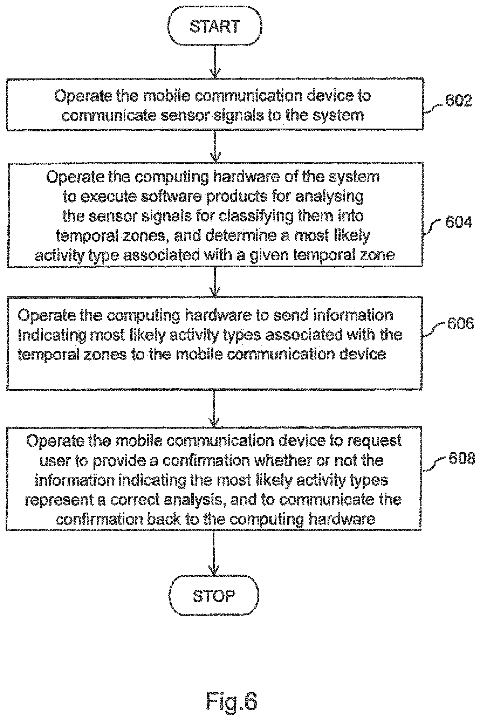

**Fig. 6** is an illustration of steps of using the system **100** for tracking and recording movements of the mobile communication device **102**, in accordance with the present disclosure, and has been explained in conjunction with Figs. 1 and 2. The method is depicted as a collection of steps in a logical flow diagram, which represents a sequence of steps that can be implemented in hardware, software, or a combination thereof.

At a step **602**, the mobile communication device **102** is operable to communicate one or more sensor signals to the server system **104**, wherein the sensor signals are indicative of motion associated with activities to which the mobile communication device **102** is exposed by its user. The sensor signals are output of one or more sensors of the mobile communication device **102**.

At a step **604**, the computing hardware of the server system **104** is operable to execute one or more software products for analyzing the sensor signals to classify them into one or more temporal zones, hereinafter referred to as activity zone, and for analyzing the sensor signals within each activity zone to determine a most likely activity type associated with the activity zone.

At a step **606**, the computing hardware is operated to send information indicating one or more most likely activity types associated with the one or more activity zones to the mobile communication device **102**.

At a step **608**, the mobile communication device **102** is operable to request its user to provide a confirmation whether or not the information indicating the most likely activity types represent a correct analysis, and to communicate the confirmation back to the computing hardware for amending parameters and/or algorithms employed in the software products which execute analysis of the sensor signals to improve their accuracy.

It should be noted here that the steps **602** to **608** are only illustrative and other alternatives can also be provided where one or more steps are added, one or more steps are removed, or one or more steps are provided in a different sequence without departing from the scope of the claims herein.

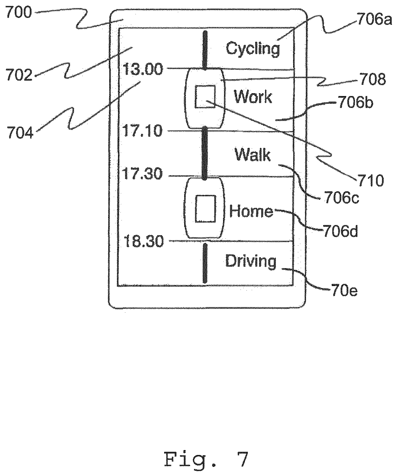

**Fig. 7** is an illustration of a graphical user interface (GUI) **702** of a mobile communication device 700, which is an example of the mobile communication device **102**, and has been explained in conjunction with Fig. 1. The embodiment illustrated in Fig. 7 is similar to the embodiment illustrated in Fig. 4. The GUI **702** displays a time-line **704** that is divided into 'activity' zones/periods **706a**, **706b**, **706c**, **706d** and **706e**, hereinafter collectively referred to as activity zones **706**, based on start and end times of one or more activities. Each activity zone **706** illustrates an activity and corresponding location of the activity. Moreover, each activity zone **706** may be illustrated by a graphical symbol **708** or a text description **710** of the corresponding activity.

In an exemplary embodiment, the timeline **704** indicates that at a time 13:00 pm, a 'cycling' activity of the user ends and he/she is stationed at a 'workplace' until a time 17:10 pm; at the time 17:10 pm, the user starts a 'walking' activity towards home; at a time 17:30 pm, the user reaches home and is at home until a time 18:30 pm; at the time 18:30 pm, the user starts a 'driving' activity.

Fig. 7 is merely an example, which should not unduly limit the scope of the claims herein. One of ordinary skill in the art would recognize many variations, alternatives, and modifications of embodiments herein.

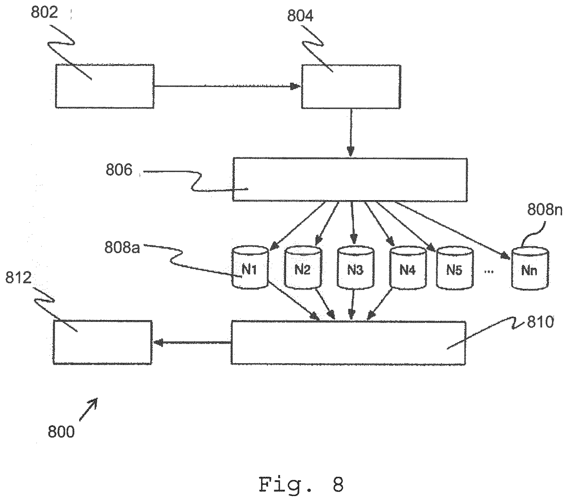

**Fig. 8** is an illustration of an activity analysis system **800**, explained in conjunction with Fig. 1, in accordance with the present disclosure. The activity analysis system **800** identify user activities based on sensor and positioning data of corresponding mobile communication device, and history, profile, demographics, and activity type of user.

In an embodiment of the present invention, the activity analysis system **800** may be present inside a remote server system **104**. In another embodiment of the present invention, the activity analysis system **800** may be present, at least in part, inside the mobile communication device **102** itself.

The activity analysis system **800** includes a receiving module **802**, a pre-processing module **804**, a pre-classification module **806**, a first through nth classifier nodes **808a** till **808n**, hereinafter referred to as classifier nodes **808**, an activity determination module **810**, and an output module **812**. The receiving module **802** collects raw data, i.e. unprocessed data from the sensors **108** and positioning systems **110** of the mobile communication device **102**. The pre-processing module **804** pre-processes the raw data collected by the receiving module **802**. Examples of pre-processing the data include, but are not limited to, filtering the data, performing domain transitions such as time to frequency domain conversion using Fast Fourier Transformation (FFT), classifying the data, averaging the data and combining the data, performing correlations with one or more pre-determined data sets representative of various types of user activities.

The pre-classification module **806** receives the pre-processed data from the pre-processing module **804** and pre-classifies it into one or more broad categories. For example, the sensor data received from a motion sensor of the mobile communication device **102** is compared with a predetermined speed value to differentiate between slow motion, i.e. walking and running stairs, and fast motion i.e. running and cycling, and classify the motion data into broad categories such as 'slow motion' and 'fast motion'. In an embodiment, the pre-classification module **806** includes rule sets and/or predefined deterministic algorithms for pre-classifying the pre-processed data.

Each classifier node **808** includes a processor that is dedicated to identifying characteristics of the sensor data corresponding to a predefined activity. For example, the first classifier node N1 **808a** may be specialized in identifying characteristics of the sensor data pertaining to 'cycling' activity, the second classifier node N2 **808b** may be specialized in identifying 'walking activity', the third classifier node N3 **808c** may be specialized in identifying 'running' activity, and so on.

The classifier nodes **808** are configured to process the pre-classified data substantially in parallel, where each classifier node **808** generates a likelihood of the corresponding predefined activity for the pre-classified data. In an exemplary embodiment, the first classifier node N1 **808a** dedicated to identification of 'cycling' activity may generate a probability value '1', the second classifier node N2 **808b** may generate a probability value '0.3' and the third classifier node N3 **808c** may generate a probability value '0.8', when the user performs a 'cycling' activity.

The activity determination module **810** may aggregate the probability values generated by the classifier nodes **808** to determine an 'activity type' corresponding to the sensor and positioning data collected by the receiving module **802**. In addition to aggregating the probabilities of the classifier nodes **808**, the activity determination module **810** may employ deterministic rules such as transition windows to determine the 'activity type'. The transition window may set inertia to activities in order not to toggle activities too often. For example, it is unlikely that an activity type would change from 'cycling' to 'walking' and back to 'cycling' very fast. The deterministic rules such as transition windows may be implemented using models such as hidden Markov models (HMM) and more complex models based on HMMs.

The output module **812** provides one or more 'activity types' determined by the activity determination module **810** to the mobile communication device **102**. In an embodiment, the output module **812** may display the determined activity types on a graphical user interface (GUI) of the mobile communication device 102 in a timeline format.

Fig. 8 is merely an example, which should not unduly limit the scope of the claims herein. One of ordinary skill in the art would recognize many variations, alternatives, and modifications of embodiments herein.

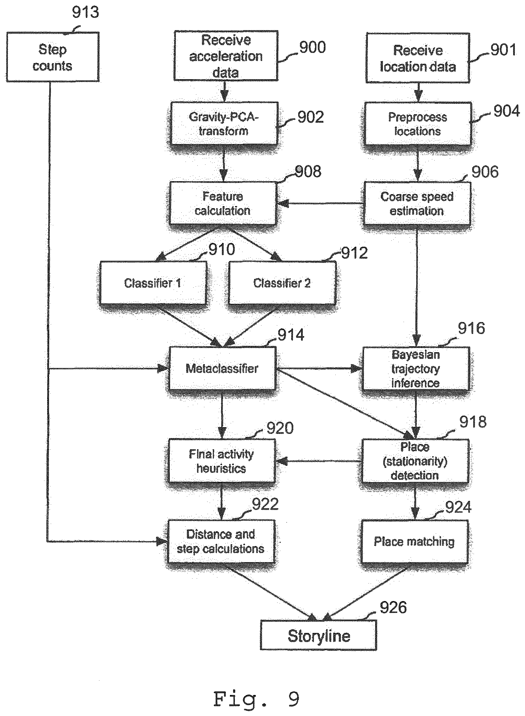

**Fig. 9** is an illustration of steps of a method for identifying a 'cycling' activity of a user based on the sensor and positioning data of the corresponding mobile communication device, in accordance with an embodiment of the present disclosure.

In an embodiment of the present invention, the steps of the method for identifying the 'cycling' activity of a user may be executed by the activity analysis system **800** of Fig. 8. The activity analysis system **800** may be present inside a remote server system 104 or may be present, at least in part, inside the mobile communication device 102 of user itself.

At a step **900**, a set of acceleration data is received from an accelerometer sensor of the mobile communication device **102**. The set of acceleration data is in raw format, i.e. unprocessed, and may be collected every 30 seconds by the accelerometer sensor. In an exemplary embodiment, the set of acceleration data has a duration of 3 seconds and includes acceleration samples collected every 1/20 seconds. Thus, each 3 second data set includes total 60 acceleration samples and each acceleration sample includes acceleration of the mobile communication device **102** in x, y, and z directions, in a coordinate system oriented with the mobile communication device **102**. Thus, each acceleration sample includes three values and the three second data set includes total 180 (3X60) values.

At a step **901**, a set of location data is received from a positioning system of the mobile communication device **102**. The positioning data may include timestamps, location coordinates, and estimated horizontal accuracy from mobile location services. In an exemplary embodiment, the location data is received at intervals ranging from few seconds to few minutes.

At a step **902**, the set of acceleration data undergoes gravity transformation, in which, for each acceleration sample, a new transformed sample is calculated, where corresponding z-component is oriented along a mean value of the acceleration vector. In another embodiment, the set of acceleration data may undergo a principal component analysis (PCA) transformation, in which, for each acceleration sample, a new transformed sample is calculated, where corresponding z-coordinate remains same, but the corresponding x and y components are transformed so that they are oriented along the principal components of the acceleration sample, when only x and y components are included.

At a step **904**, the location data may be pre-processed, where examples of pre-processing the data includes, but are not limited to, filtering the data, performing domain transitions such as time to frequency domain conversion using Fast Fourier Transformation (FFT), classifying the data, averaging the data, performing one or more correlations on the data, and combining the data. At a step **906**, a coarse speed of the mobile communication device 102 may be estimated based on the pre-processed location data. For example, the coarse speed may be estimated based on distance between consecutive location co-ordinates and time difference between the consecutive location co-ordinates.

At a step **908**, one or more features of sensor and location data are estimated based on the transformed acceleration samples and the estimated course speed, where each 'feature' has a numeric value. Examples of features include means, variances, minimums and maximums for each of the (x, y, z) components of the transformed acceleration samples, components of Fourier transformed versions of the x, y, or z components of the acceleration sample, and so forth.

The user activity corresponding to data obtained at the step **908** may be recognized based on a first classification at a step **910** using a first classifier and a second classification at a step **912** using a second classifier. In an embodiment, the first and second classifiers may be similar to classifier nodes **808** of Fig. 8. Although two classifiers are illustrated herein, it would be apparent to a person skilled in the art, that more than two classifiers can be used for recognizing the user activity.

The first and second classifiers at the steps **910** and **912** use standard supervised classification algorithms such as neural network, decision forest or support vector machine for classification. The first classifier is a binary classifier that is trained on a large training data set with training samples classified as 'cycling' or 'non-cycling', and generates an activity label 'cycling' or 'non-cycling' for the data sample obtained at the step **908**. The second classifier is a multiclass classifier that is trained on a smaller set of more accurately labeled data, and generates an activity label from one of 'cycling', 'running', 'car', 'train', 'walking' and 'other' for the data sample obtained at the step **908**.

In an embodiment, the user activity is recognized as 'cycling' if both the first and second classifiers generate activity label as 'cycling' for the data sample obtained at the step **908**. In another embodiment, the user activity is recognized based on the activity label generated by the second classifier when the first classifier generates an activity label as 'not cycling'. In yet another embodiment, the user activity is recognized as 'other', when the first classifier generates an activity label as 'not cycling' and the second classifier generates an activity label as 'cycling'. In yet another embodiment, the first classifier may generate a probability that the user activity is 'not cycling'. When the probability of 'not cycling' is high, then other classifier results indicating 'cycling' as activity might be omitted.

At a step **913**, the step counts of the user are calculated, and at a step **914**, a meta-classifier utilizes the step count data and the data generated by the first and second classifiers to combine activities recognized at the steps **910** and **912** to form one or more activity periods. In an embodiment, when there are N consecutive acceleration samples, such that 1st and last of them are labeled with a given activity, and the majority (or at least x % of them) belong to that activity, the whole period of N consecutive samples is identified as an activity period.

At a step **916**, one or more activity periods may be associated with respective locations based on location data and Bayesian 'interactive multiple models' smoothing algorithm. At a step 918, one or more stationary segments may be recognized based on the location data, when the mobile communication device **102** is stationary and no activity is performed therein.

At a step **920**, final activity heuristics-type analysis takes place based on the processed location and acceleration data. The heuristics are rules, and an example of a rule is: if a stationary segment recognized at the step **918** has a period of no recognized activity shorter than x seconds, and the neighboring activity periods have a duration greater than y seconds, the stationary segment is replaced with an activity period labeling it with the neighboring activity/activities. For example, if in a 10 minutes cycling activity, the user appears to have stopped for 1 minute in between, then the 1 minute stationary segment is ignored, and the whole 10 minutes are associated with cycling activity. Moreover, if in a 10 minutes period, there are consecutive cycling and transport activities, and there is no stopping of at least n minutes in between and no walking activity, then the whole 10 minutes period is labeled with the activity that has happened for majority of the time. However, if there is at least one detected walking sample between transport and cycling activities, then the cycling and transport activities form two different activity periods.

At a step **922**, distance and step calculations are performed, and at a step **924** place matching is performed to optimize the accuracy of user activities recognized at the step 920. Finally, at a step **926**, a storyline is created which includes various user activities in a timeline format.

Fig. 9 is merely an example, which should not unduly limit the scope of the claims herein. One of ordinary skill in the art would recognize many variations, alternatives, and modifications of embodiments herein.

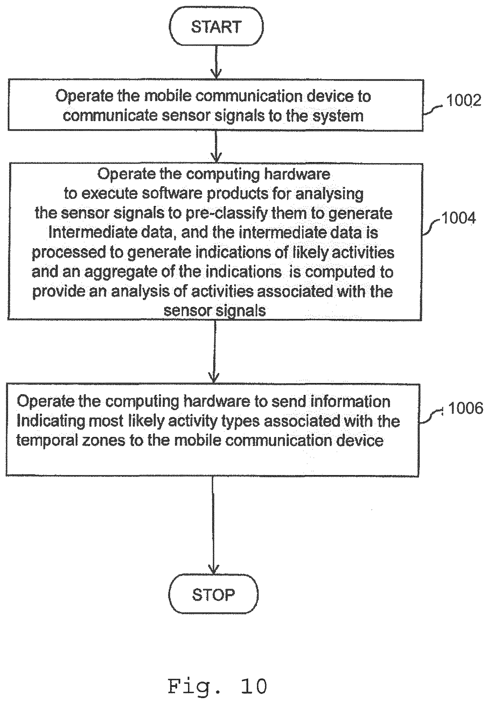

**Fig. 10** is an illustration of steps of using the system **100** for tracking and recording movements of the mobile communication device **102**, in accordance with the present disclosure, and has been explained in conjunction with Figs. 1 and 2. The method is depicted as a collection of steps in a logical flow diagram, which represents a sequence of steps that can be implemented in hardware, software, or a combination thereof.

At a step **1002**, the mobile communication device **102** is operable to communicate one or more sensor signals, or sensor data corresponding thereto, to the server system 104, wherein the sensor signals are indicative of motion associated with activities to which the mobile communication device **102** is exposed by its user. The sensor signals, or the corresponding sensor data, are outputs of one or more sensors of the mobile communication device 102.

At a step **1004**, the computing hardware of the server system **104** is operable to execute one or more software products for analyzing the sensor signals and corresponding sensor data, wherein the computing hardware is operable to pre-classify the sensor signals to generate intermediate data, and the intermediate data is thereafter processed in one or more processors to generate one or more indications of likely activities associated with the sensor signals. The computing hardware is further operable to compute an aggregate of the one or more indications to provide an analysis of activities associated with the sensor signals.

At a step **1006**, the computing hardware is operable to send information indicating most likely activity types associated with the one or more temporal zones to the mobile communication device **102**.

It should be noted here that the steps **1002** to **1006** are only illustrative and other alternatives can also be provided where one or more steps are added, one or more steps are removed, or one or more steps are provided in a different sequence without departing from the scope of the claims herein.

Although embodiments of the present invention have been described comprehensively in the foregoing, in considerable detail to elucidate the possible aspects, those skilled in the art would recognize that other versions of the invention are also possible. Embodiments of the present invention are susceptible to being employed for monitoring prisoners in prisons, for monitoring patients in home for elderly people, in hospitals and such like.

## Claims

1. A system (104) comprising:
one or more processors; and
a memory coupled to the processors comprising instructions executable by the processors, the processors being operable when executing the instructions to:
receive from a mobile communication device (102, 400, 700) one or more sensor signals indicative of motion associated with activities of a user of the mobile communication device (102, 400, 700) ;
by a pre-classification module (806, 906), select from a plurality of activity categories a particular activity category that corresponds to at least a portion of the sensor signals, wherein each of the activity categories comprises one or more activity types and is **characterized by** motion that the activity types in the activity category have in common;
by a plurality of classifier nodes (808, 910, 912), determine, by using a first analysis algorithm, from among only the activity types in the particular activity category one or more likely activity types of the user by:
analyzing the sensor signals against each of the activity types in the particular activity category; and
calculating a probability of each of the activity types of the user based on the analysis;
send the likely activity types to the mobile communication device (102, 400, 700);
request from a user of the mobile communication device (102, 400, 700) a confirmation whether or not the likely activity types represent a correct analysis, and,
in response to receiving a user confirmation from the mobile communication device (102, 400, 700), prioritize a second analysis algorithm over the first analysis algorithm, wherein the determining priority is based on determining, based on the user confirmation, that the second analysis algorithm is more accurate than the first analysis algorithm.

2. The system (104) as claimed in Claim 1, **characterized in that** :
the computing hardware of the system (104) is operable to execute one or more software products for analyzing the one or more sensor signals to classify the one or more sensor signals into one or more temporal zones (706), and to analyze the one or more sensor signals within each given temporal zone (706) to determine a most likely activity type associated with each given temporal zone (706);
wherein the computing hardware is operable to send information indicating one or more most likely activity types associated with the one or more temporal zones (706) to the mobile communication device (102, 400, 700).

3. The system (104) as claimed in Claims 1 or 2, **characterized in that** the one or more processors are configured to process the one or more sensor signals substantially in parallel, wherein the one or more processors are mutually specialized in identifying characteristics of the one or more signals corresponding to one or more activities to which the one or more processors are dedicated.

4. The system (104) as claimed in any of Claims 1 to 3, **characterized in that** determining the likely activity comprises generating a temporal log of activities experienced by the mobile communication device.

5. The system (104) as claimed in any of Claims 1 to 4, **characterized in that** the mobile communication device (102, 400, 700) comprises a personal computer, a portable media device, a smart phone, a wrist-worn phone, a phablet, a mobile telephone, or a tablet computer and/or that the mobile communication device (102, 400, 700) comprises one or more sensors comprising a gyroscopic angular sensor, an accelerometer, a GPS position sensor, a cellular position sensor, a magnetometer, a microphone, a camera, or a temperature sensor.

6. The system (104) as claimed in any of Claims 1 to 5, **characterized in that** the analysis of the sensor signals comprises:
a supervised or semisupervised classification analysis of information included in the one or more sensor signals; and
a heuristics analysis of information included in the one or more sensor signals.

7. The system (104) as claimed in Claim 6, **characterized in that** the supervised or semisupervised classification algorithms can use as input the amplitudes of the frequency components of the information included in the one or more sensor signals, and the output of the classification algorithms are estimated probabilities of different activities, conditional on the sensor signals.

8. The system (104) as claimed in any of Claims 1 to 7, **characterized in that** sending the likely activity types comprises sending analyzed activity results to the mobile computing device (102, 400, 700) for display on a graphical user interface (402, 702) of a software application of the mobile computing device (102, 400, 700), , wherein the analyzed activity results comprise a timeline (704) comprising a plurality of mutually different symbols representing different analyzed activity types.

9. A method comprising:
by a computing device (104), receiving from a mobile communication device (102, 400, 700) one or more sensor signals indicative of motion associated with activities of a user of the mobile communication device (102, 400, 700);
by a pre-classification module (806, 906) of the computing device (104), select from a plurality of activity categories a particular activity category that corresponds to at least a portion of the sensor signals, wherein each of the activity categories comprises one or more activity types and is **characterized by** motion that the activity types in the activity category have in common;
by a plurality of classifier nodes (808, 910, 912) of the computing device (104), determining, by using a first analysis algorithm, from among only the activity types in the particular activity category one or more likely activity types of the user by:
analyzing the sensor signals against each of the activity types in the particular activity category; and
calculating a probability of each of the activity types of the user based on the analysis;
by a computing device, sending the likely activity types to the mobile communication device (102, 400, 700);
by the computing device, requesting from a user of the mobile communication device (102, 400, 700) a confirmation whether or not the likely activity types represent a correct analysis, and,
in response to receiving a user confirmation from the mobile communication device (102, 400, 700), prioritizing a second analysis algorithm over the first analysis algorithm, wherein the determining priority is based on determining, based on the user confirmation, that the second analysis algorithm is more accurate than the first analysis algorithm.

10. The method of Claim 9,
wherein the sensor signals are analyzed substantially in parallel by one or more processors of the computing device (104), the processors being mutually specialized in identifying characteristics of the sensor signals corresponding to one or more activity types, and/or
wherein determining the likely activity types comprises generating a temporal log of activity types experienced by the mobile communication device.

11. The method of Claim 9,
wherein the mobile communication device (102, 400, 700) comprises a personal computer, a portable media device, a smart phone, a wrist-worn phone, a phablet, a mobile telephone, or a tablet computer and/or
wherein the mobile communication device (102, 400, 700) comprises one or more sensors comprising a gyroscopic angular sensor, an accelerometer, a GPS position sensor, cellular positioning sensor, a magnetometer, a microphone, a camera, or a temperature sensor.

12. The method of Claim 9,
wherein the analysis of the sensor signals comprises:
a supervised or semisupervised classification analysis; or
a heuristic analysis,
wherein the supervised or semisupervised classification analysis optionally comprises classification algorithms that use as input the amplitudes of the frequency components of the information included in the sensor signals, and the output of the classification algorithms optionally are estimated probabilities of different activity types based at least in part on the sensor signals.

13. The method of Claim 9, wherein sending the likely activity types comprises sending analyzed activity results to the mobile computing device for display on a graphical user interface (402, 702) of a software application of the mobile computing device (102, 400, 700), wherein the analyzed activity results comprise a timeline (704) comprising a plurality of mutually different symbols representing different analyzed activity types.

14. One or more computer-readable non-transitory storage media embodying software that is operable when executed to conduct a method according to at least one of claims 9 to 13.

15. A device (102, 400, 700) comprising:
one or more processors; and
a memory coupled to the processors comprising instructions executable by the processors, the processors being operable when executing the instructions to carry out a method including all the steps of a method according to at least one of claims 9 to 14.

## Patentansprüche

1. System, umfassend:
einen oder mehrere Prozessoren und
einen Speicher, der an die Prozessoren gekoppelt ist und Anweisungen umfasst, die durch die Prozessoren ausführbar sind, wobei die Prozessoren bei Ausführen der Anweisungen betreibbar sind zum:
Empfangen eines oder mehrerer Sensorsignale von einer Mobilkommunikationsvorrichtung (102, 400, 700), die eine Bewegung angeben, die mit Aktivitäten eines Benutzers der Mobilkommunikationsvorrichtung (102, 400, 700) assoziiert ist;
Auswählen einer bestimmten Aktivitätskategorie aus einer Mehrzahl von Aktivitätskategorien durch ein Vorklassifizierungsmodul (806, 906), die mindestens einem Teil der Sensorsignale entspricht, wobei jede der Aktivitätskategorien einen oder mehrere Aktivitätstypen umfasst und durch eine Bewegung gekennzeichnet ist, die die Aktivitätstypen in der Aktivitätskategorie gemein haben;
Bestimmen eines oder mehrerer wahrscheinlicher Aktivitätstypen des Benutzers aus nur den Aktivitätstypen in der bestimmten Aktivitätskategorie durch eine Mehrzahl von Klassifikatorknoten (808, 910, 912) durch Verwenden eines ersten Analysealgorithmus durch:
Analysieren der Sensorsignale gegen jeden der Aktivitätstypen in der bestimmten Aktivitätskategorie und
Berechnen einer Wahrscheinlichkeit von jedem der Aktivitätstypen des Benutzers auf der Basis der Analyse;
Senden der wahrscheinlichen Aktivitätstypen an die Mobilkommunikationsvorrichtung (102, 400, 700);
Anfordern einer Bestätigung, ob die wahrscheinlichen Aktivitätstypen eine korrekte Analyse darstellen oder nicht, von einem Benutzer der Mobilkommunikationsvorrichtung (102, 400, 700) und
als Reaktion auf ein Empfangen einer Benutzerbestätigung von der Mobilkommunikationsvorrichtung (102, 400, 700) Priorisieren eines zweiten Analysealgorithmus gegenüber dem ersten Analysealgorithmus, wobei die Bestimmungspriorität auf einem Bestimmen, dass der zweite Analysealgorithmus genauer als der erste Analysealgorithmus ist, auf der Basis der Benutzerbestätigung basiert.

2. System (104) nach Anspruch 1, **dadurch gekennzeichnet, dass**:
die Datenverarbeitungshardware des Systems (104) dazu betreibbar ist, ein oder mehrere Softwareprodukte zum Analysieren des einen oder der mehreren Sensorsignale auszuführen, um das eine oder die mehreren Sensorsignale in eine oder mehrere zeitliche Zonen (706) zu klassifizieren und das eine oder die mehreren Sensorsignale innerhalb jeder gegebenen zeitlichen Zone (706) zu analysieren, um einen wahrscheinlichsten Aktivitätstyp zu bestimmen, der mit jeder gegebenen zeitlichen Zone (706) assoziiert ist;
wobei die Datenverarbeitungshardware dazu betreibbar ist, eine Information, die einen oder mehrere wahrscheinlichste Aktivitätstypen angibt, die mit der einen oder den mehreren zeitlichen Zonen (706) assoziiert sind, an die Mobilkommunikationsvorrichtung (102, 400, 700) zu senden.

3. System (104) nach Anspruch 1 oder 2, **dadurch gekennzeichnet, dass** der eine oder die mehreren Prozessoren dazu konfiguriert sind, das eine oder die mehreren Sensorsignale im Wesentlichen parallel zu verarbeiten, wobei der eine oder die mehreren Prozessoren beim Identifizieren von Charakteristika des einen oder der mehreren Signale, die einer oder mehreren Aktivitäten entsprechen, für die der eine oder die mehreren Prozessoren dediziert sind, gegenseitig spezialisiert sind.

4. System (104) nach einem der Ansprüche 1 bis 3, **dadurch gekennzeichnet, dass** das Bestimmen der wahrscheinlichen Aktivität ein Erzeugen eines zeitlichen Protokolls von Aktivitäten, die von der Mobilkommunikationsvorrichtung erfahren werden, umfasst.

5. System (104) nach einem der Ansprüche 1 bis 4, **dadurch gekennzeichnet, dass** die Mobilkommunikationsvorrichtung (102, 400, 700) einen PersonalComputer, eine tragbare Medienvorrichtung, ein Smartphone, ein am Handgelenk getragenes Telefon, ein Phablet, ein Mobiltelefon oder einen Tablet-Computer umfasst und/oder dass die Mobilkommunikationsvorrichtung (102, 400, 700) einen oder mehrere Sensoren umfasst, die einen gyroskopischen Winkelsensor, einen Beschleunigungsmesser, einen GPS-Positionssensor, einen Mobilfunkpositionssensor, ein Magnetometer, ein Mikrofon, eine Kamera oder einen Temperatursensor umfassen.

6. System (104) nach einem der Ansprüche 1 bis 5, **dadurch gekennzeichnet, dass** die Analyse der Sensorsignale umfasst:
eine überwachte oder halbüberwachte Klassifizierungsanalyse einer Information, die in dem einen oder den mehreren Sensorsignalen enthalten ist; und
eine heuristische Analyse einer Information, die in dem einen oder den mehreren Sensorsignalen enthalten ist.

7. System (104) nach Anspruch 6, **dadurch gekennzeichnet, dass** die überwachten oder halbüberwachten Klassifizierungsalgorithmen die Amplituden der Frequenzkomponenten der Information, die in dem einen oder den mehreren Sensorsignalen enthalten ist, als Eingabe verwenden können und es sich bei der Ausgabe der Klassifizierungsalgorithmen um geschätzte Wahrscheinlichkeiten von unterschiedlichen Aktivitäten in Abhängigkeit von den Sensorsignalen handelt.

8. System (104) nach einem der Ansprüche 1 bis 7, **dadurch gekennzeichnet, dass** das Senden der wahrscheinlichen Aktivitätstypen ein Senden von analysierten Aktivitätsergebnissen an die Mobildatenverarbeitungsvorrichtung (102, 400, 700) zur Anzeige auf einer grafischen Benutzeroberfläche (402, 702) einer Softwareanwendung der Mobildatenverarbeitungsvorrichtung (102, 400, 700) umfasst, wobei die analysierten Aktivitätsergebnisse eine Zeitleiste (704) umfassen, die eine Mehrzahl von voneinander verschiedenen Symbolen umfasst, die unterschiedliche analysierte Aktivitätstypen darstellen.

9. Verfahren, umfassend:
Empfangen eines oder mehrerer Sensorsignale von einer Mobilkommunikationsvorrichtung (102, 400, 700) durch eine Datenverarbeitungsvorrichtung (104), die eine Bewegung angeben, die mit Aktivitäten eines Benutzers der Mobilkommunikationsvorrichtung (102, 400, 700) assoziiert ist;
Auswählen einer bestimmten Aktivitätskategorie aus einer Mehrzahl von Aktivitätskategorien durch ein Vorklassifizierungsmodul (806, 906) der Datenverarbeitungsvorrichtung (104), die mindestens einem Teil der Sensorsignale entspricht, wobei jede der Aktivitätskategorien einen oder mehrere Aktivitätstypen umfasst und durch eine Bewegung gekennzeichnet ist, die die Aktivitätstypen in der Aktivitätskategorie gemein haben;
Bestimmen eines oder mehrerer wahrscheinlicher Aktivitätstypen des Benutzers aus nur den Aktivitätstypen in der bestimmten Aktivitätskategorie durch eine Mehrzahl von Klassifikatorknoten (808, 910, 912) der Datenverarbeitungsvorrichtung (104) durch Verwenden eines ersten Analysealgorithmus durch:
Analysieren der Sensorsignale gegen jeden der Aktivitätstypen in der bestimmten Aktivitätskategorie und
Berechnen einer Wahrscheinlichkeit von jedem der Aktivitätstypen des Benutzers auf der Basis der Analyse;
Senden der wahrscheinlichen Aktivitätstypen durch eine Datenverarbeitungsvorrichtung an die Mobilkommunikationsvorrichtung (102, 400, 700);
Anfordern einer Bestätigung, ob die wahrscheinlichen Aktivitätstypen eine korrekte Analyse darstellen oder nicht, von einem Benutzer der Mobilkommunikationsvorrichtung (102, 400, 700) durch die Datenverarbeitungsvorrichtung und
als Reaktion auf ein Empfangen einer Benutzerbestätigung von der Mobilkommunikationsvorrichtung (102, 400, 700) Priorisieren eines zweiten Analysealgorithmus gegenüber dem ersten Analysealgorithmus, wobei die Bestimmungspriorität auf einem Bestimmen, dass der zweite Analysealgorithmus genauer als der erste Analysealgorithmus ist, auf der Basis der Benutzerbestätigung basiert.

10. Verfahren nach Anspruch 9,
wobei die Sensorsignale durch einen oder mehrere Prozessoren der Datenverarbeitungsvorrichtung (104) im Wesentlichen parallel analysiert werden, wobei die Prozessoren beim Identifizieren von Charakteristika der Sensorsignale, die einer oder mehreren Aktivitäten entsprechen, gegenseitig spezialisiert sind, und/oder
wobei das Bestimmen der wahrscheinlichen Aktivität ein Erzeugen eines zeitlichen Protokolls von Aktivitätstypen, die von der Mobilkommunikationsvorrichtung erfahren werden, umfasst.

11. Verfahren nach Anspruch 9,
wobei die Mobilkommunikationsvorrichtung (102, 400, 700) einen PersonalComputer, eine tragbare Medienvorrichtung, ein Smartphone, ein am Handgelenk getragenes Telefon, ein Phablet, ein Mobiltelefon oder einen Tablet-Computer umfasst und/oder
wobei die Mobilkommunikationsvorrichtung (102, 400, 700) einen oder mehrere Sensoren umfasst, die einen gyroskopischen Winkelsensor, einen Beschleunigungsmesser, einen GPS-Positionssensor, einen Mobilfunkpositionssensor, ein Magnetometer, ein Mikrofon, eine Kamera oder einen Temperatursensor umfassen.

12. Verfahren nach Anspruch 9,
wobei die Analyse der Sensorsignale umfasst:
eine überwachte oder halbüberwachte Klassifizierungsanalyse oder
eine heuristische Analyse,
wobei die überwachte oder halbüberwachte Klassifizierungsanalyse optional Klassifizierungsalgorithmen umfasst, die die Amplituden der Frequenzkomponenten der Information, die in den Sensorsignalen enthalten ist, als Eingabe verwenden, und es sich bei der Ausgabe der Klassifizierungsalgorithmen optional um geschätzte Wahrscheinlichkeiten von unterschiedlichen Aktivitätstypen zumindest zum Teil auf der Basis der Sensorsignale handelt.

13. Verfahren nach Anspruch 9, wobei das Senden der wahrscheinlichen Aktivitätstypen ein Senden von analysierten Aktivitätsergebnissen an die Mobildatenverarbeitungsvorrichtung (102, 400, 700) zur Anzeige auf einer grafischen Benutzeroberfläche (402, 702) einer Softwareanwendung der Mobildatenverarbeitungsvorrichtung (102, 400, 700) umfasst, wobei die analysierten Aktivitätsergebnisse eine Zeitleiste (704) umfassen, die eine Mehrzahl von voneinander verschiedenen Symbolen umfasst, die unterschiedliche analysierte Aktivitätstypen darstellen.

14. Ein oder mehrere computerlesbare nichtflüchtige Speichermedien, die Software verkörpern und die dazu betreibbar sind, bei Ausführen ein Verfahren nach mindestens einem der Ansprüche 9 bis 13 zu leiten.

15. Vorrichtung (102, 400, 700), umfassend:
einen oder mehrere Prozessoren und
einen Speicher, der an die Prozessoren gekoppelt ist und Anweisungen umfasst, die durch die Prozessoren ausführbar sind, wobei die Prozessoren dazu betreibbar sind, bei Ausführen der Anweisungen ein Verfahren durchzuführen, das alle Schritte eines Verfahrens nach mindestens einem der Ansprüche 9 bis 14 umfasst.

## Revendications

1. Système (104) comprenant:
un ou plusieurs processeurs; et
une mémoire couplée aux processeurs comprenant des instructions exécutables par les processeurs, les processeurs étant aptes, lors de l'exécution des instructions, à:
recevoir, à partir d'un dispositif de communication mobile (102, 400, 700), un ou plusieurs signaux de capteur indiquant un déplacement associé à des activités d'un utilisateur du dispositif de communication mobile (102, 400, 700);
par un module de pré-classification (806, 906), sélectionner, à partir d'une pluralité de catégories d'activités, une catégorie d'activités particulière qui correspond à au moins une partie des signaux de capteur, chacune des catégories d'activités comprenant un ou plusieurs types d'activités et étant **caractérisée par** un déplacement que les types d'activités dans la catégorie d'activités ont en commun;
par une pluralité de noeuds de classificateur (808, 910, 912), déterminer, à l'aide d'un premier algorithme d'analyse, parmi uniquement les types d'activités dans la catégorie d'activités particulière, un ou plusieurs types d'activités probables de l'utilisateur par:
analyse des signaux de capteur par rapport à chacun des types d'activités dans la catégorie d'activités particulière; et
calcul d'une probabilité de chacun des types d'activités de l'utilisateur sur la base de l'analyse;
envoyer les types d'activités probables au dispositif de communication mobile (102, 400, 700);
demander à un utilisateur du dispositif de communication mobile (102, 400, 700) une confirmation du point de savoir si les types d'activités probables représentent ou non une analyse correcte, et
en réponse à la réception d'une confirmation d'utilisateur à partir du dispositif de communication mobile (102, 400, 700), donner la priorité à un second algorithme d'analyse plutôt qu'au premier algorithme d'analyse, la détermination de priorité étant basée sur la détermination, sur la base de la confirmation d'utilisateur, du fait que le second algorithme d'analyse est plus précis que le premier algorithme d'analyse.

2. Système (104) tel que revendiqué à la revendication 1, **caractérisé en ce que**:
le matériel informatique du système (104) est apte à exécuter un ou plusieurs produits logiciels pour analyser le ou les signaux de capteur pour classer le ou les signaux de capteur dans une ou plusieurs zones temporelles (706), et pour analyser le ou les signaux de capteur dans chaque zone temporelle (706) donnée pour déterminer un type d'activités le plus probable associé à chaque zone temporelle (706) donnée;
le matériel informatique étant apte à envoyer des informations indiquant un ou plusieurs types d'activités les plus probables associés à la ou aux zones temporelles (706) au dispositif de communication mobile (102, 400, 700).

3. Système (104) tel que revendiqué à la revendication 1 ou 2, **caractérisé en ce que** le ou les processeurs sont configurés pour traiter le ou les signaux de capteur sensiblement en parallèle, le ou les processeurs étant mutuellement spécialisés dans l'identification de caractéristiques du ou des signaux correspondant à une ou plusieurs activités auxquelles le ou les processeurs sont dédiés.

4. Système (104) tel que revendiqué à l'une quelconque des revendications 1 à 3, **caractérisé en ce que** la détermination de l'activité probable comprend la génération d'un journal temporel d'activités subies par le dispositif de communication mobile.

5. Système (104) tel que revendiqué à l'une quelconque des revendications 1 à 4, **caractérisé en ce que** le dispositif de communication mobile (102, 400, 700) comprend un ordinateur personnel, un dispositif multimédia portable, un téléphone intelligent, un téléphone porté au poignet, une phablette, un téléphone mobile ou une tablette électronique et/ou **en ce que** le dispositif de communication mobile (102, 400, 700) comprend un ou plusieurs capteurs comprenant un capteur angulaire gyroscopique, un accéléromètre, un capteur de position GPS, un capteur de position cellulaire, un magnétomètre, un microphone, une caméra ou un capteur de température.

6. Système (104) tel que revendiqué à l'une quelconque des revendications 1 à 5, **caractérisé en ce que** l'analyse des signaux de capteur comprend:
une analyse de classification supervisée ou semi-supervisée d'informations incluses dans le ou les signaux de capteur; et
une analyse heuristique d'informations incluses dans le ou les signaux de capteur.

7. Système (104) tel que revendiqué à la revendication 6, **caractérisé en ce que** les algorithmes de classification supervisée ou semi-supervisée peuvent utiliser comme entrées les amplitudes des composantes de fréquence des informations incluses dans le ou les signaux de capteur, et les sorties des algorithmes de classification sont des probabilités estimées de différentes activités, conditionnées par les signaux de capteur.

8. Système (104) tel que revendiqué à l'une quelconque des revendications 1 à 7, **caractérisé en ce que** l'envoi des types d'activités probables comprend l'envoi de résultats d'activités analysées au dispositif informatique mobile (102, 400, 700) pour un affichage sur une interface utilisateur graphique (402, 702) d'une application logicielle du dispositif informatique mobile (102, 400, 700), les résultats d'activités analysées comprenant une ligne temporelle (704) comprenant une pluralité de symboles mutuellement différents représentant différents types d'activités analysés.

9. Procédé comprenant:
par un dispositif informatique (104), recevoir à partir d'un dispositif de communication mobile (102, 400, 700) un ou plusieurs signaux de capteur indiquant un déplacement associé à des activités d'un utilisateur du dispositif de communication mobile (102, 400, 700);
par un module de pré-classification (806, 906) du dispositif informatique (104), sélectionner, à partir d'une pluralité de catégories d'activités, une catégorie d'activités particulière qui correspond à au moins une partie des signaux de capteur, chacune des catégories d'activités comprenant un ou plusieurs types d'activités et étant **caractérisée par** un déplacement que les types d'activités dans la catégorie d'activités ont en commun;
par une pluralité de noeuds de classificateur (808, 910, 912) du dispositif informatique (104), déterminer, à l'aide d'un premier algorithme d'analyse,
parmi uniquement les types d'activités dans la catégorie d'activités particulière, un ou plusieurs types d'activités probables de l'utilisateur par:
analyse des signaux de capteur par rapport à chacun des types d'activités dans la catégorie d'activités particulière; et
calcul d'une probabilité de chacun des types d'activités de l'utilisateur sur la base de l'analyse;
par un dispositif informatique, envoyer les types d'activités probables au dispositif de communication mobile (102, 400, 700);
par le dispositif informatique, demander à un utilisateur du dispositif de communication mobile (102, 400, 700) une confirmation du point de savoir si les types d'activités probables représentent ou non une analyse correcte, et en réponse à la réception d'une confirmation d'utilisateur à partir du dispositif de communication mobile (102, 400, 700), donner la priorité à un second algorithme d'analyse plutôt qu'au premier algorithme d'analyse, la détermination de priorité étant basée sur la détermination, sur la base de la confirmation d'utilisateur, du fait que le second algorithme d'analyse est plus précis que le premier algorithme d'analyse.

10. Procédé selon la revendication 9,
dans lequel les signaux de capteur sont analysés sensiblement en parallèle par un ou plusieurs processeurs du dispositif informatique (104), les processeurs étant mutuellement spécialisés dans l'identification de caractéristiques des signaux de capteur correspondant à un ou plusieurs types d'activités, et/ou
dans lequel la détermination des types d'activités probables comprend la génération d'un journal temporel de types d'activités subis par le dispositif de communication mobile.

11. Procédé selon la revendication 9,
dans lequel le dispositif de communication mobile (102, 400, 700) comprend un ordinateur personnel, un dispositif multimédia portable, un téléphone intelligent, un téléphone porté au poignet, une phablette, un téléphone mobile ou une tablette électronique, et/ou
dans lequel le dispositif de communication mobile (102, 400, 700) comprend un ou plusieurs capteurs comprenant un capteur angulaire gyroscopique, un accéléromètre, un capteur de position GPS, un capteur de position cellulaire, un magnétomètre, un microphone, une caméra ou un capteur de température.

12. Procédé selon la revendication 9,
dans lequel l'analyse des signaux de capteur comprend:
une analyse de classification supervisée ou semi-supervisée; ou
une analyse heuristique,
l'analyse de classification supervisée ou semi-supervisée comprenant de manière facultative des algorithmes de classification qui utilisent comme entrées les amplitudes des composantes de fréquence des informations incluses dans les signaux de capteur, et les sorties des algorithmes de classification sont de manière facultative des probabilités estimées de différents types d'activités basées au moins en partie sur les signaux de capteur.

13. Procédé selon la revendication 9, dans lequel l'envoi des types d'activités probables comprend l'envoi de résultats d'activités analysées au dispositif informatique mobile pour un affichage sur une interface utilisateur graphique (402, 702) d'une application logicielle du dispositif informatique mobile (102, 400, 700), les résultats d'activités analysées comprenant une ligne temporelle (704) comprenant une pluralité de symboles mutuellement différents représentant différents types d'activités analysés.

14. Support(s) de stockage non transitoire(s) lisible(s) par ordinateur incorporant un logiciel qui est apte, lorsqu'il est exécuté, à réaliser un procédé selon au moins l'une des revendications 9 à 13.

15. Dispositif (102, 400, 700) comprenant:
un ou plusieurs processeurs; et
une mémoire couplée aux processeurs comprenant des instructions exécutables par les processeurs, les processeurs étant aptes, lors de l'exécution des instructions, à réaliser un procédé comprenant toutes les étapes d'un procédé selon au moins l'une des revendications 9 à 14.
